(19)

Europäisches
Patentamt
European
Patent Office
Office européen
des brevets

(11) **EP 3 613 405 A1**

(12) **EUROPEAN PATENT APPLICATION**
published in accordance with Art. 153(4) EPC

(43) Date of publication:
**26.02.2020 Bulletin 2020/09**

(51) Int Cl.:
*A61K 8/02* (2006.01)　　*A45D 40/00* (2006.01)
*A61K 8/34* (2006.01)　　*A61K 8/35* (2006.01)
*A61K 8/81* (2006.01)　　*A61K 8/84* (2006.01)
*A61L 26/00* (2006.01)　　*A61Q 3/00* (2006.01)
*A61Q 19/00* (2006.01)　　*B05B 5/025* (2006.01)
*B05B 11/00* (2006.01)　　*B05D 1/04* (2006.01)

(21) Application number: 18787460.7

(22) Date of filing: 19.04.2018

(86) International application number:
**PCT/JP2018/016186**

(87) International publication number:
**WO 2018/194132 (25.10.2018 Gazette 2018/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 19.04.2017　JP 2017082841
19.04.2017　JP 2017083248
19.04.2017　JP 2017083249

(71) Applicant: Kao Corporation
**Chuo-Ku
Tokyo 103-8210 (JP)**

(72) Inventors:
• **MUKAI, Kenta
Haga-gun
Tochigi 321-3497 (JP)**
• **TOHJO, Takehiko
Haga-gun
Tochigi 321-3497 (JP)**
• **FUKUDA, Ikuo
Haga-gun
Tochigi 321-3497 (JP)**

(74) Representative: **Hoffmann Eitle
Patent- und Rechtsanwälte PartmbB
Arabellastraße 30
81925 München (DE)**

(54) **METHOD FOR FORMING COATING FILM**

(57)　　Provided is a method for forming a coating comprising fibers having an excellent adhesivity to the skin, comprising directly electrostatically spraying on the skin.

A method for producing a coating comprising fibers on the surface of skin or nail, comprising directly electrostatically spraying onto human skin or nail, composition A comprising the following components (a), (b) and (c) :
(a) one or more volatile substances selected from the group consisting of an alcohol and a ketone,
(b) a water-insoluble polymer for fiber formation,
(c) 0.2 mass% or more and 25 mass% or less of water; wherein the mass ratio of component (b) to component (c), ((b)/(c)) is of 0.4 or more and 50 or less.

[Figure 7]

**Description**

[Field of the Invention]

The present invention relates to a method for producing a coating by electrostatic spraying.

[Background of the Invention]

Various methods are known for forming a coating containing fibers by electrostatic spraying. For example, in Patent Literature 1, a method using a portable electrospinning device is disclosed, but there is no specific description of the solvents for injecting a liquefied polymer. Patent Literature 2 discloses a method for forming a discontinuous film on the skin by electrostatic spraying, but the discontinuous film obtained is a liquid coating containing particles, but not a coating containing fibers. Patent Literature 3 discloses a method for forming fibers by direct electrostatic spraying on humans. As a specific example, a composition in which polyvinyl alcohol is dissolved in a water-ethanol solution is disclosed, but since polyvinyl alcohol is water-soluble, when it comes to directly spraying on the skin, conditions such as spraying in a high-temperature environment are required to dry the composition, and the composition is sprayed as droplets, so that it is difficult to form a fibrous film. Furthermore, for water-soluble fibers, the resulting coating may be dissolved in perspiration.
(Patent Literature 1) JP-A-2004-525272
(Patent Literature 2) JP-A-2006-95332
(Patent Literature 3) JP-A-2007-32534

[Summary of the Invention]

The present invention relates to a method for producing a coating comprising fibers on the skin or nail, comprising directly electrostatically spraying composition A onto a human skin or nail, wherein the composition A comprises the following components (a), (b) and (c):

(a) one or more volatile substances selected from the group consisting of an alcohol and a ketone,
(b) a water-insoluble polymer for fiber formation,
(c) 0.2 mass% or more and 25 mass% or less of water,

wherein the mass ratio of component (b) to component (c), ((b)/(c)), is 0.4 or more and 50 or less.
The present invention further relates to a composition for use in producing a coating comprising fibers on a human skin or nail by direct electrostatic spraying of the composition onto a human skin or nail by means of an electrostatic spraying device holdable by a human hand or an electrostatic spraying device having an operation unit comprising a spraying nozzle holdable by a human hand, wherein the following components (a), (b) and (c):

(a) a volatile substance comprising ethanol,
(b) a water-insoluble polymer for fiber formation,
(c) from 0.2 mass% to 25 mass% of water,

wherein the mass ratio of component (b) to component (c), ((b)/(c)), is 0.4 or more and 50 or less.

[Brief Description of the Drawings]

FIG. 1 is a schematic view showing a configuration of an electrostatic spraying device suitably used in the present invention.
FIG. 2 is a schematic view showing the configuration of another embodiment of an electrostatic spraying device suitable for use in the present invention.
FIG. 3 is a schematic view showing a configuration of a cartridge portion and a main body portion provided in the electrostatic spraying device shown in FIG. 2.
FIG. 4 is a schematic view showing another embodiment of a cartridge portion and a main body portion provided in the electrostatic spraying device shown in FIG. 2.

FIG. 5 is a schematic view showing a configuration of still another embodiment of an electrostatic spraying device used in the present invention.

FIG. 6 is a schematic view showing a structure of a cartridge portion provided in the electrostatic spraying device shown in FIG. 5.

FIG. 7 is a schematic view showing a state in which an electrostatic spraying method is performed by using an electrostatic spraying device.

[Detailed Description of the Invention]

When the water-insoluble polymer is directly coated on the skin by electrostatic spraying, even if the coating is formed, the coating with fibers cannot be formed and lacks flexibility, or even if a coating containing fibers is obtained, the coating has a problem of low adhesivity to the skin.

Thus, the present invention relates to a method for forming a coating with good adhesivity and fibers by direct electrostatic spraying on the skin.

As a result of extensive studies on the composition to be electrostatically sprayed, the present inventors found that if a water-insoluble polymer is used as a coating-forming polymer and a certain amount of water is contained in addition to a volatile substance such as an alcohol or a ketone, a film containing fibers having good adhesivity to the skin etc. can be stably formed when the composition is electrostatically sprayed directly onto the surface of skin or nail, thereby completing the present invention.

According to the method for producing a coating according to the present invention, it is possible to form a coating with fibers of moisture insoluble polymers by electrostatic spraying, while it is possible to improve the conductivity by the presence of water, and it is possible to spray fibers and water simultaneously, and it is possible to improve the adhesivity of the fibers to the skin.

In the present invention, composition A (hereinafter also referred to as spraying composition A) for direct electrostatic spraying of human skin or nail to form a coating containing fibers on the skin or nail contains the following components (a), (b) and (c):

(a) one or more volatile substances selected from the group consisting of an alcohol and a ketone,
(b) a water-insoluble polymers for fiber formation, and
(c) 0.2 mass% or more and 25 mass% or less of water,

wherein the mass ratio of component (b) to component (c), ((b)/(c)), is 0.4 or more and 50 or less.

The (a) volatile substance is a substance having volatility in a liquid state. In the spraying composition, component (a) is discharged from the nozzle tip toward the skin after the spraying composition A placed in the electric field is sufficiently charged, and when component (a) evaporates, the charge density of the spraying composition A becomes excessive, and the component (a) evaporates further while being further refined by Coulomb repulsion, and finally, component (a) is blended for the purpose of forming a coating containing dry fibers. For this purpose, the volatile substance preferably has a vapor pressure at 20°C of 0.01 kPa or more and 106.66 kPa or less, more preferably 0.13 kPa or more and 66.66 kPa or less, even more preferably 0.67 kPa or more and 40.00 kPa or less, and even more preferably 1.33 kPa or more and 40.00 kPa or less.

Among the (a) volatile substance, monovalent chain aliphatic alcohols, monovalent cyclic aliphatic alcohols, monovalent aromatic alcohols, for example, are suitably used as alcohols. Examples of the monovalent chain aliphatic alcohol include $C_1$-$C_6$ alcohol, $C_4$-$C_6$ cyclic alcohol as the monovalent cyclic alcohol, and benzyl alcohol, phenylethyl alcohol, and the like as the monovalent chain aliphatic alcohol. Specific examples thereof include ethanol, isopropyl alcohol, butyl alcohol, phenylethyl alcohol, n-propanol, n-pentanol. As these alcohols, one or more kinds selected therefrom can be used.

Among the (a) volatile substance, examples of the ketone include a dialkyl ketone $C_1$-$C_4$, for example, acetone, methyl ethyl ketone, methyl isobutyl ketone. These ketones can be used alone or in combination of two or more.

The (a) volatile substance is preferably one or more selected from the group consisting of ethanol, isopropyl alcohol and butyl alcohol, more preferably one or two selected from the group consisting of ethanol and butyl alcohol; even more preferably a volatile substance containing ethanol, from the viewpoint of the feel of the formed fiber.

The content of component (a) in the spraying composition A is preferably 50 mass% or more, more preferably 55 mass% or more, further more preferably 60 mass% or more, and even more preferably 65 mass% or more. The content is also preferably 95 mass% or less, more preferably 94 mass% or less, further more preferably 93 mass% or less, and even more preferably 92 mass% or less. The content of component (a) in the spraying composition A is preferably 50 mass% or more and 95 mass% or less, more preferably 55 mass% or more and 94 mass% or less, further more preferably 60 mass% or more and 93 mass% or less, even more preferably 65 mass% or more and 92 mass% or less, and further even more preferably 60 mass% or more and 92 mass% or less. By containing component (a) in the spraying composition A at this ratio, the spraying composition A can be sufficiently volatilized when the electrostatic spraying method is performed, and a coating containing fibers can be formed on the surface of the skin or the nail.

From the viewpoint of the volatility of the volatile substance and the feel of the fiber to be formed, the content of ethanol is preferably 50 mass% or more, more preferably 65 mass% or more, and more preferably 80 mass% or more, based on the total amount of the (a) volatile substance. It is preferable that the content of ethanol be 100 mass % or less. The content of ethanol is preferably 50 mass% or more and 100 mass% or less, more preferably 65 mass% or more and 100 mass% or less, and even more preferably 80 mass% or more and 100 mass% or less, based on the total amount of the (a) volatile substance.

The (b) water-insoluble polymer for fiber formation is a substance that can be dissolved in the (a) volatile substance. Here, dissolving means a dispersed state at 20°C, and the dispersed state is a visually uniform state, preferably a visually transparent or translucent state.

As the water-insoluble polymer for fiber formation, a polymer suitable for the nature of the (a) volatile substance may be used. Specifically, the polymer is soluble in component (a) and insoluble in water. As used herein, the term "water-soluble polymer" means a polymer having a property in which, after weighing the polymer 1 g at 1 atm and at 23°C, the polymer is immersed in 10 g of ion-exchanged water, and 0.5 g or more of the immersed polymer is dissolved in water after a lapse of 24 hours. The "water-insoluble polymer" in the present specification refers to a polymer having a property that 0.5 g or more of the polymer immersed in 10 g of ion-exchanged water is not dissolved after 24 hours after weighing the polymer 1 g under the atmosphere of 1 atm and 23°C; in other words, it has the dissolution property of less than 0.5 g.

Examples of a polymer having a fiber formability which is insoluble in water include a fully saponified polyvinyl alcohol which can be insolubilized after a coating is formed, a partially saponified polyvinyl alcohol which can be crosslinked by using in combination with a crosslinking agent after a coating is formed, an oxazoline-modified silicone such as poly(N-propanoylethyleneimine)graft-dimethylsiloxane/γ-aminopropyl methylsiloxane copolymer, polyvinyl acetal diethylaminoacetate, Zein (a main component of corn protein), polyester, polylactic acid (PLA), acrylic resin (e.g., a polyacrylonitrile resin, and a polymethacrylic acid resin), a polystyrene resin, a polyvinyl butyral resin, a polyethylene terephthalate resin, a polybutylene terephthalate resin, a polyurethane resin, a polyamide resin, a polyimide resin, a polyamide-imide resin. These water-insoluble polymers can be used alone or in combination of two or more. Among these water-insoluble polymers, it is preferable to use one or more selected from the group consisting of a fully saponified polyvinyl alcohol which can be insolubilized after the formation of a coating, a partially saponified polyvinyl alcohol which can be crosslinked after the formation of a coating by using in combination with a crosslinking agent, a polyvinyl butyral resin, a polyurethane resin, an acrylic acid resin such as a polymethacrylate resin, an oxazoline-modified silicone (e.g., polyvinyl-acetal diethylaminoacetate, poly(N-propanoylethyleneimine) graft-dimethylsiloxane/γ-aminopropylmethylsiloxane copolymer), polylactic acid (PLA), and Zein.

Among these water-insoluble polymer, a partially saponified polyvinyl alcohol, a fully saponified polyvinyl alcohol, a polyvinyl butyral resin, a polymethacrylic resin, and a polyurethane resin are more preferable, a partially saponified polyvinyl alcohol, a fully saponified polyvinyl alcohol, and a polyvinyl butyral resin are more preferable, and a polyvinyl butyral resin is more preferable, from the viewpoint of stable and efficient formation of a coating containing fibers on the surface of skin or nail, coating durability, coating formability, and compatibility with the skin and coating durability.

The content of component (b) in the spraying composition A is preferably 1 mass% or more, more preferably 2 mass% or more, even more preferably 3 mass% or more, even more preferably 5 mass% or more, and even more preferably 7 mass% or more. Further, 30 mass% or less is preferable, 25 mass% or less is more preferable, 20 mass% or less is more preferable, and 17 mass% or less is even more preferable . The content of component (b) of A in the compositions for spraying is preferably 1 mass% or more and 30 mass% or less, more preferably 2 mass% or more and 25 mass% or less, even more preferably 3 mass% or more and 20 mass% or less, even more preferably 5 mass% or more and 20 mass% or less, and even more preferably 7 mass% or more and 17 mass% or less. By containing component (b) in the spraying composition at this ratio, a coating of fibers can be formed stably and efficiently.

Component (c) is water. Since water is ionized and charged as compared with a non-ionized solvent such as ethanol, the spraying composition A can be given conductivity. Therefore, a coating of fibers is stably formed on the surface of the skin and the nail by electrostatic spraying. Water also contributes to improved adhesivity of the coating formed by electrostatic spraying onto the skin and nail, improved coating durability, and appearance. From the viewpoint of obtaining these effects, the composition (c) contains 0.2 mass% or more and 25 mass% or less in the spraying composition A. The content of the component (c) in the spraying composition A is preferably 0.3 mass% or more, more preferably 0.35 mass% or more, and even more preferably 0.4 mass% or more. The content of component (c) in the spraying composition A is preferably 20 mass% or less, more preferably 19 mass% or less, more preferably 18 mass% or less, and is preferably 10 mass% or less from the viewpoint of ensuring the formability of the coating of fibers even in summer and humid environments. The content of water in the compositions for spraying A is 0.2 mass% or more and 25 mass% or less, 0.3 mass% or more and 20 mass% or less, 0.35 mass% or more and 19 mass% or less, 0.4 mass% or more and 18 mass% or less, and even more preferably 0.4% mass or more and 10 mass% or less from the viewpoint of formability of the coating of fibers even in humid environments.

In addition, the mass ratio of the component (b) to the component (c), ((b)/(c)), is 0.4 or more and 50 or less from the viewpoint of forming a coating of fibers on the surface of skin or nail, improving the coating adhesivity to the skin etc. , and improving coating durability. The mass ratio ((b)/(c)) is preferably 0.5 or more, and more preferably 0.6 or more. The mass ratio ((b)/(c)) is preferably 45 or less, and more preferably 40 or less. The mass ratio ((b)/(c)) is from 0.4 to 50, preferably from 0.5 to 45, more preferably from 0.55 to 40, and even more preferably from 0.6 to 40.

The mass ratio of component (a) to component (c), ((a)/(c)), is preferably 3 or more and 300 or less, from the viewpoint of obtaining a coatingof fibers stably by direct electrostatic spraying of the spraying composition A, improvement in coating adhesivity, improvement in coating durability, and the like. The mass ratio ((a)/(c)) is more preferably 3.5 or more, and more preferably 4 or more. The mass ratio ((a)/(c)) is more preferably 250 or less, and more preferably 210 or less. The mass ratio ((a)/(c)) is more preferably 3.5 or more and 250 or less, and more preferably 4 or more and 210 or less.

When the component (a) is ethanol, similarly, the mass ratio of component (a) to component (c), ((a)/(c)), is preferably 3 or more and 300 or less, more preferably 3.5 or more and 250 or less, and even more preferably 4 or more and 210 or less.

The ratio of the content of component (b) to ethanol (a) in the spraying composition A, ((b)/(a)), is preferably 0.02 or more and 0.6 or less, preferably 0.025 or more and 0.4 or less, further more preferably 0.04 or more and 0.3 or less, even more preferably 0.07 or more and 0.3 or less, further even more preferably 0.08 or more and 0.25 or less, from the viewpoint of sufficiently volatilizing component (a) such as ethanol when performing the electrostatic spraying method and stably forming the coating of fibers. When component (a) contains ethanol, the mass ratio ((b)/(a)) is preferably 0.02 or more and 0.6 or less, more preferably 0.025 or more and 0.4 or less, further more preferably 0.04 or more and 0.3 or less, even more preferably 0.07 or more and 0.3 or less, and particularly preferably 0.08 or more and 0.25 or less.

The spraying composition A may contain only the above components (a) to (c), or may contain other components in addition to the components (a) to (c). Examples of the other components include a polyol, a liquid oil, a plasticizer of the (b) polymer, a conductivity controlling agent of spraying composition A, a water-soluble polymer, a powder (e.g., acoloringpigment, a constitutive pigment), adye, aperfume, arepellent, an antioxidant, a stabilizer, a preservative, various vitamins. When the spraying composition contains other component(s), the content ratio of the other components is preferably 0.1 mass% or more and 30 mass% or less, and more preferably 0.5 mass% or more and 20 mass% or less.

In addition, the spraying composition may contain glycol. Examples of the glycol include ethylene glycol, propylene glycol, butylene glycol, diethylene glycol, dipropylene glycol, polypropylene glycol, and the like. From the viewpoint of sufficiently volatilizing the component (a) when performing the electrostatic spraying method, from the viewpoint of fiber formability, and from the viewpoint of the feel of the fibers formed, the content of the glycol in composition A is preferably 10 mass% or less, more preferably 3 mass% or less, further more preferably 1 mass% or less, and the spraying composition is preferably substantially free of glycol.

The spraying composition A may contain powders such as a coloring pigment and a constitutive pigment, but the content of the powder having a particle diameter of 0.1 $\mu$m or more at 20°C is preferably 1 mass% or less, more preferably 0.1 mass% or less, further more preferably 0.01 mass% or less, from the viewpoint of uniform coating formability, coating durability and coating adhesivity, and it is preferable not to contain a powder except when inevitably mixed in.

The viscosity of the spraying composition A at 25°C is preferably from 2 to 3,000 mPa·s from the viewpoint of stably forming a coating of fibers, the viewpoint of spinnability at the time of electrostatic spraying, the viewpoint of improving coating durability, and the viewpoint of improving the feel of the coating. The viscosity is preferably 10 mPa·s or more, more preferably 15 mPa·s or more, even more preferably 20 mPa·s or more, and even more preferably 30 mPa·s or more. The viscosity is preferably 1,500 mPa·s or less, more preferably 1,000 mPa·s or less, and even more preferably 800 mPa·s or less. The viscosity range is preferably 2 mPa·s or more and 3000 mPa·s or less, more preferably 10 mPa·s or more and 1,500 mPa·s or less, even more preferably 15 mPa·s or more and 1,000 mPa·s or less, and even more preferably 15 mPa·s or more and 800 mPa·s or less. The viscosity of the spraying composition A is measured at 25°C using a type E viscometer. As the E-type viscometer, for example, an E-type viscometer (VISCONIC EMD) manufactured by Tokyo Meters Co., Ltd. can be used. Then, the measurement conditions are 25°C, rotor No.43 of the cone plate, and an appropriate rotation speed corresponding to the viscosity is selected, and the viscosity of 500 mPa·S or more is 5 rpm, the viscosity of 150 mPa·S or more and less than 500 mPa·S is 10 rpm, and the viscosity of less than 150 mPa·S is 20 rpm.

Among the spraying composition A as described above, it is preferable to contain the following components (a), (b) and (c):

    (a) a volatile substance containing ethanol,
    (b) a water-insoluble polymer for fiber formation,

(c) 0.2% to 25 mass% of water,

wherein a mass ratio of component (b) to component (c), ((b)/(c)), is 0.4 or more and 50 or less, from the viewpoint of stably forming a coating of fibers, spinnability at the time of electrostatic spraying, and coating durability.

Here, as the (b) water-insoluble polymer for forming the fiber, as described above, one or more of water-insoluble polymers selected from the group consisting of a fully saponified polyvinyl alcohol, a partially saponified polyvinyl alcohol, a polyvinyl butyral resin, a polyurethane resin, and a polymethacrylic acid resin are preferable, more preferably a water-insoluble polymer selected from the group consisting of a polyvinyl butyral resin and a polyurethane resin, further more preferably a polyvinyl butyral resin, and even more preferably a water-insoluble resin at least containing polyvinyl butyral resin.

In the present invention, the spraying composition A is electrostatically sprayed directly onto the human skin or nail to form a coating containing fibers on the skin or nail. More specifically, the present invention relates to a method of forming a coating of fibers on the surface of human skin or nail by spraying the spraying composition A with an electrostatic spraying device, preferably an electrostatic spraying device holdable by a human hand, or an electrostatic spraying device that has an operation unit provided with a spraying nozzle holdable by a human hand.

In the present invention, the target object to be electrostatically sprayed onto is human skin or nail.

The spraying composition A is sprayed directly onto the area of the human skin or nail to be coated by an electrostatic spraying method. The electrostatic spraying method involves electrostatically spraying the spraying composition onto the skin by using an electrostatic spraying device. The electrostatic spraying device basically includes a container containing the composition, a nozzle for discharging the composition therefrom, a supply device for supplying the composition contained in the container to the nozzle, and a power supply for applying a voltage to the nozzle.

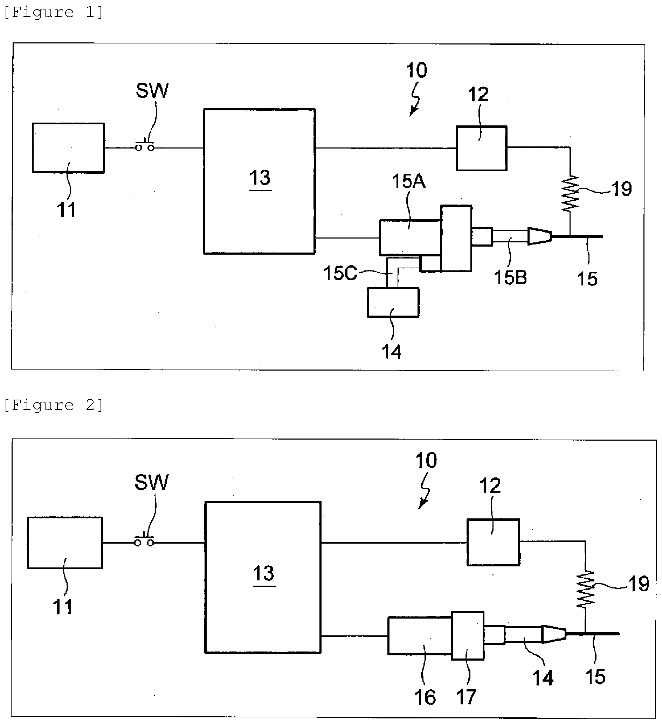

FIG. 1 is a schematic view showing a configuration of an electrostatic spraying device preferably used in the present invention. The electrostatic spraying device 10 shown in the figure includes a low-voltage power supply 11. The low-voltage power supply 11 can generate a voltage of several V up to 20 V. For the purpose of enhancing the portability of the electrostatic spraying device 10, the low-voltage power supply 11 preferably comprises one or more batteries. Further, by using a battery as the low-voltage power supply 11, there is an advantage that replacement can be easily performed as necessary. Instead of the battery, an AC adapter or the like can be used as the low-voltage power supply 11.

The electrostatic spraying device 10 also includes a high-voltage power supply 12. The high-voltage power supply 12 is connected to the low-voltage power supply 11, and includes an electric circuit (not shown) for boosting a voltage generated by the low-voltage power supply 11 to a high voltage. The booster electric circuit is generally composed of a transformer, a capacitor, a semiconductor element, and the like.

The electrostatic spraying device 10 further comprises an auxiliary electrical circuit 13. The auxiliary electric circuit 13 is interposed between the low-voltage power supply 11 and the high-voltage power supply 12, and has a function of adjusting the voltage of the low-voltage power supply 11 to stably operate the high-voltage power supply 12. Further, the auxiliary electric circuit 13 has a function of controlling the number of revolutions of a motor provided in a microgear pump 15A, which will be described hereafter. By controlling the rotation speed of the motor, the supply amount of the spraying composition from the container 15 of the spraying composition described hereafter to the microgear pump 15A is controlled. A switch SW is mounted between the auxiliary electric circuit 13 and the low-voltage power supply 11 such that the electrostatic spraying device 10 can be turned on and off by turning the switch SW on and off.

The electrostatic spraying device 10 further comprises a nozzle 15. The nozzle 15 is made of a conductive material such as metal or a non-conductive material such as plastic, rubber, or ceramic provided around the conductive material, and has a shape capable of discharging the spraying composition from the tip thereof. A minute space through which the spraying composition flows is formed in the nozzle 15 along the longitudinal direction of the nozzle 15. The size of the cross section of the micro space is preferably 100 $\mu$m or more and 2,000 $\mu$m or less in terms of diameter, and preferably 300 $\mu$m or more and 1,400 $\mu$m or less. From the same viewpoint, the flow path length of the nozzle 15 is preferably 2 mm or more and 25 mm or less, and more preferably 4 mm or more and 15 mm or less.

The nozzle 15 communicates with a microgear pump 15A via a pipeline 15B. The conduit 15 B may be conductive or non-conductive. The nozzle 15 is electrically connected to the high-voltage power supply 12. This makes it possible to apply a high voltage to the nozzle 15. In this case, in order to prevent an excessive current from flowing when a human body directly touches the nozzle 15, the nozzle 15 and the high voltage power supply 12 are electrically connected via a current-limiting resistor 19. The nozzle 15 and the high-voltage power supply 12 may be electrically connected to each other via a conductor such as an electrode (not shown).

The microgear pump 15A communicating with the nozzle 15 via the conduit 15B functions as a supply device for supplying the nozzle 15 with the spraying composition contained in the container 14. The microgear pump 15A is operated by receiving a power supply from the low-voltage power supply 11. The microgear pump 15A is configured to supply a predetermined amount of the spraying composition to the nozzle 15 under the control of the auxiliary electric circuit 13.

The container 14 is connected to the microgear pump 15A via a flexible pipe 15C. Contained in the container 14 is the spraying composition. The container 14 preferably has a cartridge-type replaceable configuration. More preferably, the cartridge is a refillable cartridge. The cartridge is preferably filled with the spraying composition A in a refillable cartridge.

In light of compatibility between the handling property of the electrostatic spraying device 10 and good discharge of the spraying composition, the volume of the accommodating portion 15 is preferably 1mL or more, more preferably 1.5 mL or more, more preferably 3mL or more, more preferably 25 mL or less, more preferably 20mL or less, and even more preferably 15 mL or less. Specifically, the volume of the accommodation portion 15 is preferably 1mL or more and 25 mL or less, more preferably 1.5 mL or more and 20mL or less, and even more preferably 3 mL or more and 15 mL or less. Such a range of the volume of the container 15 (also referred to as an accommodating portion 15) is advantageous in that the container 15 is small and can be replaced more easily when the container 15 is in a cartridge-type replaceable form.

Another embodiment of an electrostatic spraying device for use in the present invention will now be described with respect to FIGS. 2 to 6. The electronic spraying device 10 shown in FIGS. 2 to 6 differs from the electronic spraying device shown in Figure 1 in that it has a castle portion and a body portion. With respect to the embodiment shown in FIGS. 2 to 6, the description of the embodiment shown in FIG. 1 is appropriately applied to the point that the description is not particularly given. In FIGS. 2 to 6, the same members as those in FIG. 1 are denoted by the same reference numerals.

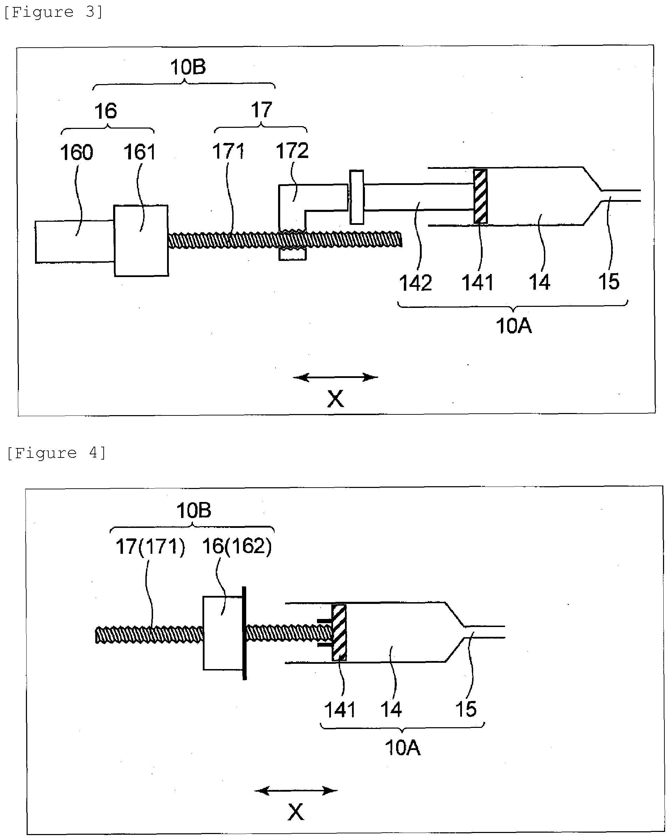

The electrostatic spraying device 10 of the embodiment shown in FIGS. 2 and 3 roughly includes a cartridge portion 10A and a main body portion10B. The cartridge portion 10A includes an accommodating portion 14, a gasket 141, a plunger 142, and a nozzle 15. The main body portion 10B includes a low voltage power supply 11, a high-voltage power supply 12, an auxiliary electric circuit 13, a power source 16 including a DC motor 160 and a motor reduction gear 161, and a power transmission portion 17 including a screw shaft 171 and a feed screw 172.

The cartridge portion 10A is detachable from the main body portion 10B. As a result, according to the electrostatic spraying device 10 of the present embodiment, the spraying composition can be easily refilled, and the cleanliness of the nozzle 15 can be maintained. Examples of the form in which the cartridge portion 10A is detachable from the main body portion 10B include, but are not limited to, a method in which a fitting portion is formed and fitted to the plunger 142 and the feed screw 172, and a method in which a thread is formed and screwed to the plunger 142 and the feed screw 172.

As shown in FIG. 3, the cartridge portion 10A includes a cylindrical accommodating portion 14 capable of accommodating the spraying composition. One end of the accommodating portion 14 forms an opening. A nozzle 15 is provided at the other end of the accommodating portion 14. A gasket 141 is disposed inside the accommodating portion 14. The gasket 141 has a shape having the same contour as the shape of the cross section of the accommodating portion 14. As a result, the gasket 141 is in liquid-tight contact with the inner surface of the accommodating portion 14, and is slidable within the accommodating portion 14. The gasket 141 is coupled to the front end of the plunger 142. The plunger 142 extends beyond the opening of the accommodating portion 14 to the outside of the accommodating portion 14. The rear end of the plunger 142 engages the lead screw 172. The accommodating portion 14 may be conductive or non-conductive.

The nozzle 15 in the present embodiment communicates with the container 14. The nozzle 15 is electrically connected to the high-voltage power supply 12. This makes it possible to apply a high voltage to the nozzle 15. In this case, in order to prevent an excessive current from flowing when a human body directly touches the nozzle 15, the nozzle 15 and the high-voltage power supply 12 are electrically connected via a current-limiting resistor 19. The nozzle 15 and the high-voltage power supply 12 may be electrically connected to each other via a conductor such as an electrode (not shown).

As described above, the cartridge portion 10A in the electrostatic spraying device 10 of the present embodiment is described below with respect to the main body portion 10B of the present embodiment.

The main body 10B includes a low voltage power supply 11. The low-voltage power supply 11 can generate a voltage of several V to 20 V. For the purpose of enhancing the portability of the electrostatic spraying device 10, the low voltage power supply 11 preferably comprises one or more batteries. Further, by using a battery as the low voltage power supply 11, there is an advantage that replacement can be easily performed as necessary. Instead of the battery, an AC adapter or the like can be used as the low-voltage power supply 11.

The main body 10B further includes a high-voltage power supply 12. The high-voltage power supply 12 is connected to the low voltage power supply 11, and includes an electric circuit (not shown) for boosting a voltage generated by the low-voltage power supply 11 to a high voltage. The booster electric circuit is generally composed of a transformer, a capacitor, a semiconductor element, and the like.

The main body 10B further includes an auxiliary electrical circuit 13. The auxiliary electric circuit 13 is interposed between the above low-voltage power supply 11 and the high-voltage power supply 12, and the auxiliary electric circuit 13 has a function of adjusting the voltage of the low-voltage power supply 11 to stably operate the high-voltage power supply 12. Further, the auxiliary electric circuit 13 has a function of controlling the number of revolutions of a motor provided in power source 16, which will be described hereafter. By controlling the number of revolutions of the motor, the supply amount of the spraying composition to the nozzle 15 for discharging the spraying composition from the accommodating portion 14 in which the spraying composition is accommodated is controlled. A switch SW is mounted between the auxiliary electric circuit 13 and the low-voltage power supply 11 such that the electrostatic spraying device 10 can be turned on and off by turning the switch SW on and off.

The main body portion 10B further includes a power source 16 and a power transmission portion 17. The power source 16 generates power for discharging the spraying composition contained in the accommodating portion 14 from the nozzle 15. The power source 16 is supplied with power from the low-voltage power source 11, and operates under the control of the auxiliary electric circuit 13. The power generated from the power source 16 is transmitted to the cartridge portion 10A by the power transmission portion 17, and a predetermined amount of the spraying composition can be supplied from the accommodating portion 14 to the nozzle 15.

The main body 10B has a power source 16 with a direct-current motor 160 and a motor reducer 161, and a power transmission unit 17 with a screw shaft 171 and a feed screw 172.

The direct-current motor 160 is linked to the motor reducer 161, which is output by reducing the revolving power of the direct-current motor 160. The motor reducer 161 is linked to a screw shaft 171. The revolving power generated by the direct-current motor 160 is transmitted to the screw shaft 171 through the motor reducer 161 and the screw shaft 171 turns. The rotational power transmitted to the screw shaft 171 is converted into linear power for moving the position of the feed screw 172 along the longitudinal direction X by the feed screw 172 connected to the screw shaft 171. The linearly moving feed screw 172 pushes the plunger 142 and gasket 141 into the accommodating portion 14, thereby allowing the spraying composition accommodated in the accommodating portion 14 to be sprayed outwardly through the nozzle 15.

In FIG. 3, the feed screw 172 and the plunger 142 are separated and the plunger 142 and the gasket 141 are integrated, but the feed screw 172 and the plunger 142 may be integrated and the plunger 142 and the gasket 141 may be separated, or the feed screw 172 and the gasket 141 may be in direct contact and transmit linear power to the gasket 141.

Due to the conductivity of the spraying composition used in the present invention, high voltages applied to the nozzles 15 may be applied to the power source 16 via the spraying composition or the power transmission unit 17 accommodated in the accommodating portion 14, and the electrostatic spraying device 10 may be damaged or leaked and not sprayed. Therefore, it is preferable that the power transmission unit 17 electrically insulate the power source 16 and the cartridge unit 10A from each other from the viewpoint of ensuring the insulating property at the time of use of the electrostatic spraying device 10 and preventing the electrostatic spraying device 10 from being damaged, and from the viewpoint of preventing the current from leaking through the spraying composition and ensuring the spinning stability. This effectively prevents the high voltage applied to the nozzle 15 from being unintentionally applied to the power source 16. As a method of electrically isolating the power source 16 and the cartridge portion 10A, for example, a method of using a screw shaft 171 and a feed screw 172 made of a non-conductive material such as plastic or ceramic as the power transmission portion 17 can be cited. If the power transmission unit 17 electrically insulates the power source 16 from the cartridge unit 10A, the gasket 141 and the plunger 142 maybe electrically conductive or non-conductive.

FIG. 4 shows another embodiment of an electrostatic spraying device 10 suitable for use in the present invention. With respect to the embodiment shown in FIG. 4, the description of the embodiment shown in FIG. 2 and FIG. 3 is appropriately applied to the points that are not particularly described. In FIG. 4, the same members as those in FIG. 2 and FIG. 3 are denoted by the same reference numerals.

As shown in FIG. 4, the cartridge portion 10A includes an accommodating portion 14, a gasket 141, and a nozzle 15. The main body 10B includes a power source 16 having a linear step motor 162 and a power transmission unit 17 having a screw shaft 171. With these configurations, the supply amount of the spraying composition from the accommodating portion 14 to the nozzle 15 can be precisely controlled, and the portability of the electrostatic spraying device 10 can be further enhanced due to the miniaturization of the main body portion 10B.

The linear stepper motor 162 has a rotor (not shown) with an internal female thread. A screw shaft 171, which is an external thread, is screwed to the female thread of the rotor. The linear stepping motor 162 is fixed to a frame (not shown) of the main body 10B. The rotation of the rotor in the linear stepping motor 162 causes the screw shaft 171 screwed to the female thread of the rotor to move linearly along the longitudinal direction X. The linearly moving screw shaft 171 pushes the gasket 141 in contact with one end of the screw shaft 171 into the accommodating portion 14, thereby allowing the spraying composition accommodated in the accommodating portion 14 to be sprayed to the outside through the nozzle 15.

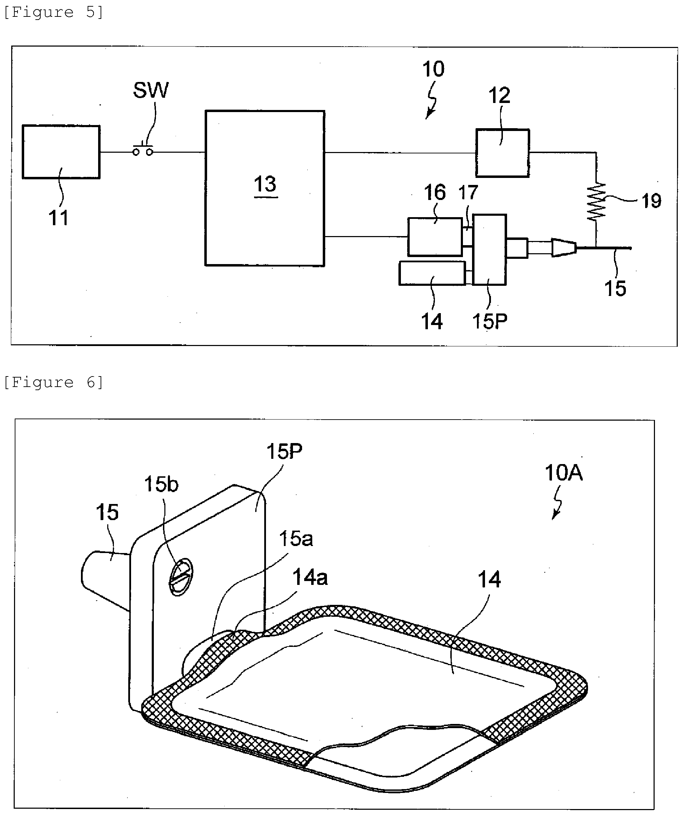

FIG. 5 shows a further embodiment of an electrostatic spraying device . The electrostatic spraying device 10 shown in the figure roughly includes a cartridge portion 10A and a main body portion 10B. The cartridge portion 10A includes an accommodating portion 14, a nozzle 15, and a pump portion 15P. The main body 10B includes a low voltage power supply 11, a high-voltage power supply 12, an auxiliary electric circuit 13, a power source 16, and a power transmission unit 17.

The cartridge portion 10A is detachable from the main body portion 10B. As a result, according to the electrostatic spraying device 10 of the present embodiment, the spraying composition can be easily refilled, and the cleanliness of the nozzle 15 can be maintained. As a form in which the cartridge portion 10A is detachable from the main body portion 10B, for example, a method in which a fitting portion is formed in a power transmission portion 17, which will be described hereafter, and the pump portion 15P and the power source 16 are fitted via the power transmission portion 17 can be cited, but the method is not limited to these methods.

The accommodating portion 14 is capable of accommodating the spraying composition, and is detachable from the cartridge portion 10A. As a result, according to the electrostatic spraying device 10 of the present embodiment, refilling of the spraying composition can be performed more easily. As a form in which the accommodating portion 14 is detachable from the cartridge portion 10A, for example, a method in which an engaging portion is formed and engaged with the accommodating portion 14 and the pump portion 15P can be cited, but the present invention is not limited to these methods.

FIG. 6 shows the cartridge portion 10A in the present embodiment. The cartridge portion 10A includes a bag-shaped accommodating portion 14. The accommodating portion 14 is formed in a flat bag shape by superposing two sheets made of a liquid impermeable and flexible material such as polyethylene and joining their outer edges in a liquid-tight manner. Thereby, the accommodating portion 14 has a deformable structure, and the spraying composition can be accommodated inside the accommodating portion 14. The accommodating portion 14 has an opening 14a at a part of its outer edge which can communicate with the pump portion 15P for supplying the spraying composition to the pump portion 15P. The accommodating portion 14 may be a conductive material or a non-conductive material.

The nozzle 15 in the present embodiment communicates with the pump portion 15P. The nozzle 15 is electrically connected to the high-voltage power supply 12. This makes it possible to apply a high voltage to the nozzle 15. In this case, in order to prevent an excessive current from flowing when a human body directly touches the nozzle 15, the nozzle 15 and the high-voltage power supply 12 are electrically connected via Current-limiting resistor 19. The nozzle 15 and the high-voltage power supply 12 may be electrically connected to each other via a conductor such as an electrode (not shown). The nozzle 15 may further include a conduit for communicating with the pump portion 15P. The conduit may be conductive or non-conductive.

The cartridge portion 10A includes a pump portion 15P. The pump portion 15P is interposed between the accommodating portion 14 and the nozzle 15 and communicates with each other. The pump portion 15P has a connection portion 15a with the opening portion 14a of the accommodating portion 14. This makes it possible to supply the spraying composition accommodated in the accommodating portion 14 to the nozzle 15 while maintaining the liquid-tightness of the flow path between the accommodating portion 14 and the pump portion 15P. The pump unit 15P is driven by the power of a power source 16, which will be described hereafter, being transmitted via the power transmission unit 17. The pump unit 15P shown in FIG. 6 is further provided with a rotatable power transmission recess 15b for transmitting the power of the power source 16 to an internal mechanism such as a gear (not shown) in the pump unit 15P via the power transmission unit 17. As the pump unit 15P, a gear pump or the like is used from the viewpoint of improving the discharge quantitative property of the spraying composition and the viewpoint of improving the portability of the electrostatic spraying device 10. As the gear pump, for example, a microgear pump or the like described in the embodiment shown in FIG. 1 can be used.

When the spraying composition is discharged from the accommodating portion 14 to the nozzle 15 by the operation of the pump portion 15P due to the liquid-tightness of the flow path between the accommodating portion 14 and the pump portion 15P being maintained, the spraying composition in the accommodating portion 14 decreases along with the supply to the nozzle 15, and the internal pressure in the accommodating portion 14 decreases. As a result, the accommodating portion 14 made of a flexible and deformable material is deformed so as to collapse in response to the internal pressure.

As described above, the cartridge portion 10A in the electrostatic spraying device 10 of the present embodiment is described below with respect to the main body portion 10B of the present embodiment.

The main body 10B includes a low voltage power supply 11. The low-voltage power supply 11 can generate a voltage of several V to 20 V. For the purpose of enhancing the portability of the electrostatic spraying device 10, the low voltage power supply 11 preferably comprises one or more batteries. Further, by using a battery as the low voltage power supply 11, there is an advantage that replacement can be easily performed as necessary. Instead of the battery, an AC adapter or the like can be used as the low-voltage power supply 11.

The main body 10B includes a high-voltage power supply 12. The high-voltage power supply 12 is connected to the low-voltage power supply 11, and includes an electric circuit (not shown) for boosting a voltage generated by the low-voltage power supply 11 to a high voltage. The booster electric circuit is generally composed of a transformer, a capacitor, a semiconductor element, and the like.

The body 10B comprises an auxiliary electrical circuit 13. The auxiliary electric circuit 13 is interposed between the low-voltage power supply 11 and the high-voltage power supply 12, and has a function of adjusting the voltage of the low-voltage power supply 11 to stably operate the high-voltage power supply 12. Further, the auxiliary electric circuit 13 has a function of controlling the number of revolutions of a motor provided in power source 16, which will be described hereafter. By controlling the number of revolutions of the motor, the supply amount of the spraying composition to the nozzle 15 for discharging the spraying composition from the accommodating portion 14 in which the spraying composition is accommodated through the pump portion 15P is controlled. A switch SW is mounted between the auxiliary electric circuit 13 and the low-voltage power supply 11 such that the electrostatic spraying device 10 can be turned on and off by turning the switch SW on and off.

The main body portion 10B further includes a power source 16 and a power transmission portion 17. The power source 17 of the present embodiment generates power for discharging the spraying composition contained in the accommodating portion 14 from the nozzle 15 via the pump portion 15P. The power source 16 in the present invention is provided with a motor or the like, and is supplied with power from the low-voltage power supply 11 and operated under the control of the supplementary electric circuits 13 to generate power. The power generated from the power source 16 is configured such that a predetermined amount of the spraying composition can be supplied from the accommodating portion 14 to the nozzle 15 via the pump portion 15P by the power transmission portion 17 which transmits the power to the pump portion 15P.

The power transmission section 17 provided in the main body section 10B transmits power generated from a power source 16 such as a motor to the pump section 15P. As a result, the spraying composition contained in the accommodating portion 14 can be supplied to the nozzle 15 via the pump portion 15P. The method of transmitting the power from the power source 16 to the pump portion 15P is not particularly limited, but the rotational power of the motor in the power source 16 can be transmitted to the pump portion 15P, for example, by forming a convex structure (not shown) at the tip of the power transmission portion 17 formed of a shaft and fitting the convex structure to the power transmission concave portion 15b provided in the pump portion 15P.

Due to the conductivity of the spraying compositions used in the present invention, high voltages applied to the nozzles 15 are applied to the power source 16 via the pumping section 15P and the power transmission section 17, and the electrostatic spraying device 10 may be damaged or may leak charges and not be sprayed. Therefore, it is preferable that the power transmission unit 17 electrically insulate the power source 16 and the cartridge unit 10A from each other from the viewpoint of ensuring the insulating property at the time of use of the electrostatic spraying device 10 and preventing the electrostatic spraying device 10 from being damaged, and from the viewpoint of preventing the current from leaking through the spraying composition and ensuring the spinning stability. This effectively prevents the high voltage applied to the nozzle 15 from being unintentionally applied to the power source 16. Examples of the method of electrically insulating the power source 16 and the cartridge portion 10A include a method of using a member made of a non-conductive material such as plastic or ceramic as the power transmission portion 17. As long as the power transmission unit 17 electrically insulates the power source 16 from the cartridge unit 10A, the other constituent materials may be conductive or non-conductive.

The above configuration enables to precisely control the supply amount of the spraying composition from the accommodating portion 14 to the nozzle 15, and further enhance the portability of the electrostatic spraying device 10, due to the miniaturization of the main body portion 10B.

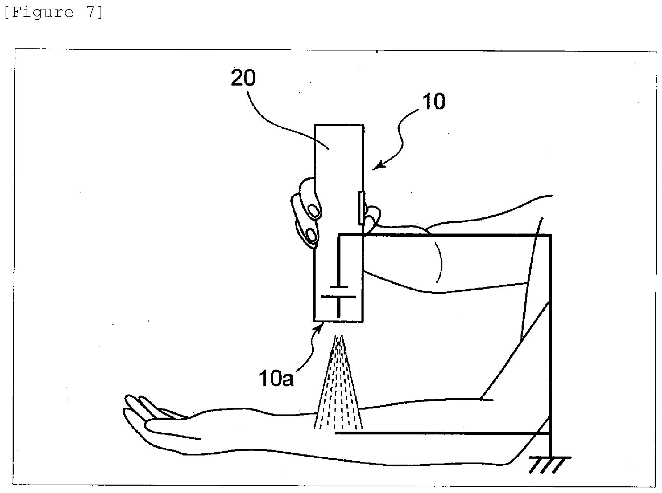

The electrostatic spraying device 10 having the configuration shown in the embodiments shown in FIGS. 1 to 6 can be used, for example, as shown in FIG. 7. FIG. 7 shows an electrostatic spraying device 10 of the hand-held type having a size holdable by one hand. The electronic spraying device 10 shown in this figure, all the members of the configuration figures shown in FIGS. 1, 2 or 5 are housed in a cylindrically-shaped housing 20. Although the housing 20 shown in FIG. 7 is cylindrical, the shape of the housing 20 is not particularly limited as long as the housing 20 has a size holdable by one hand and all of the members shown in FIG. 1 are accommodated, and the housing 20 may have a cylindrical shape such as an elliptical cylindrical shape or a regular polygonal cylindrical shape. The "size holdable by one hand" means that the weight of the housing 20 accommodating the electrostatic spraying device 10 is preferably 2 kg or less, the maximum longitudinal length of the housing 20 is preferably 40 cm or less, and the volume of the housing 20 is preferably 3000 cm$^3$ or less. A nozzle, not shown, is disposed at one longitudinal end 10a of the housing 20. The nozzle is disposed in the housing 20 such that the blowing direction of the spraying composition coincides with the longitudinal direction of the housing 20 and is convex toward the skin side. By arranging the nozzle tip so as to be convex toward the skin in the longitudinal direction of the housing 20, the spraying composition hardly adheres to the housing, and the coating can be stably formed.

Next explains a case where a coating is formed on the surface of skin or nail.

When the user operates the electrostatic spraying device 10, the user, i.e., the person who forms a coating on the skin or nail by the electrostatic spraying, holds the device 10 by his/her hand and directs one end 10a of the device 10, where a nozzle (not shown) is located, to the site to be sprayed electrostatically. In FIG. 7, one end 10a of the electrostatic spraying device 10 is shown pointing inside the forearm of the user. In this state, the device 10 is switched on to perform the electrostatic spraying method. When the device 10 is powered on, an electric field is generated between the nozzle and the skin. In the embodiment shown in FIG. 7, a positive high voltage is applied to the nozzle and the skin becomes the negative electrode. When an electric field is generated between the nozzle and the skin, the spraying composition at the tip of the nozzle is polarized by electrostatic induction such that the tip portion becomes conical, and droplets of the spraying composition charged from the tip of the cone are discharged into the air atmosphere toward the skin along the electric field. When the component (a) as a solvent evaporates from the spraying composition discharged into the space (atmosphere) and charged, the charge density on the surface of the spraying composition becomes excessive, and spreads into the space by repeated refinement by Coulomb repulsion force, and reaches the skin. In this case, by appropriately adjusting the viscosity of the spraying composition, the sprayed composition can reach the application site in the form of droplets. Alternatively, while being discharged into the space, a volatile substance (a solvent), may be volatilized from a droplet to solidify a polymer, which is a solute and has a coating formability, while the fiber is formed while being stretched and deformed by a potential difference, and the fiber may be deposited at an application site. For example, increasing the viscosity of the spraying composition facilitates deposition of the composition in the form of fibers at the site of application. This forms a porous coating of a fiber deposit on the surface of the application site. A porous coating of such a fiber deposit can also be formed by adjusting the distance between the nozzle and the skin and the voltage applied to the nozzle.

During the electrostatic spraying, a high potential difference is generated between the nozzle and the skin. However, because of very large impedance, the current extremely hardly flows through the human body. For example, the present inventors have confirmed that the current flowing through the human body during the electrostatic spraying process is several orders of magnitude smaller than the current flowing through the human body due to static electricity generated during normal living.

When a fiber deposit is formed by the electrostatic spraying method, the thickness of the fiber is preferably 10 nm or more, more preferably 50 nm or more, when expressed as a circle equivalent diameter. The thickness is preferably 3,000 nm or less, and more preferably 1,000 nm or less. The thickness of the fiber can be measured by observing the fiber at a magnification of $10,000 \times$ by, for example, scanning electron microscopy (SEM) observation, removing defects (clumps of fibers, crossing portions of fibers, and droplets) from the two-dimensional image, arbitrarily selecting 10 fibers, drawing a line orthogonal to the longitudinal direction of the fiber, and directly reading the fiber diameter.

The coating, which is a fiber deposit formed by the electrostatic spraying method, has a coating film in which moisture is present on the surface side of the constituent fibers. Surface side of fiber means a surface, or a part of a surface, or a place between fibers. When the water content of the spraying composition is approximately 0.2 mass % or more and the mass ratio of the polymer (b) having a fiber formability to the water (c), ((b)/(c)), is 0.4 or more, a coating film in which water is supported is easily formed on the surface of the constituent fibers or between the fibers, and the transparency and adhesivity of the fibers can be improved. Meanwhile, if the water content in the spraying composition is less than 0.2 mass %, and the mass ratio of the polymer (b) having a fiber formability to the water (c), ((b)/(c)), is less than 0.4, the moisture bearing coating is hardly formed on the surfaces of the constituent fibers, which affects the coating durability and coating adhesivity. In addition, when the moisture-bearing coating is formed on the fibers constituting the coating in this manner, the adhesivity with the skin increases, the coating tends to become transparent, and the coating gets closer to a natural appearance. Further, since the adhesivity increases, it is effective in moisturizing the skin or improving the skin condition. The effect of the adhesivity is further enhanced by the application of the liquid agent before or after the electrostatic spraying, which will be described hereafter.

In the case of skin containing sweat, sebum, and the like, moisture and oil components are compounded in the fibers, so that the fibers tend to swell or plasticize. For example, when the same composition is electrostatically sprayed on a metal surface containing no moisture or oil and on a skin surface containing moisture or oil, for example, on the palm of the hand, for 5 seconds to prepare a thin film, when a change in the fiber diameter is observed over time, the fiber electrostatically sprayed on the skin surface becomes larger in diameter over time by swelling than the fiber electrostatically sprayed on the metal surface. Thus, the fiber-containing coating formed by electrostatic spraying is plasticized by the oil and moisture in the skin to become softer, thereby improving the followability of the fiber itself to the skin texture, and the moisture bleeds out from the fiber and exists between the fiber surface and the fiber, whereby the fiber-containing coating becomes translucent or transparent, thereby imparting a natural appearance. When the object to be coated is a skin containing sweat, sebum, or the like, the fiber diameter due to swelling satisfies the following equation (1):

```
(Fiber diameter 30 seconds after spinning on the skin) > (Fiber diameter
30 seconds after spinning on the metal plate) (1)
```

The contents of the components (a), (b) and (c) which is the spraying composition are measured as follows. Since the component (a) which is a volatile substance does not exist in the formed film or volatilizes even if it exists, the formed film is measured in a state in which only the component (b) and the component (c) are contained, and the content thereof is measured as follows.

<Determination of the Content of Component (b) and Component (c) of the spraying composition>

Separate identification by liquid chromatography (HPLC) and identification by infrared spectrophotometer (IR) are available in solution. Since the liquid chromatograph elutes from a component having a large molecular weight, the composition can be identified by predicting the molecular weight or by determining the elution position of component. It is also possible to assign and identify functional groups from individual absorbers by IR analysis, and it is generally possible to identify them by comparing IR charts of components with standard charts of commercial additives.

<Determination of the Content of Component (b) and Component (c) in the formed coating>

A solvent capable of dissolving the coating film is searched for, and after dissolving the coating film in the solvent, separation/identification by a liquid chromatograph (HPLC) or identification by an infrared spectrophotometer (IR) is performed.

The fiber forming the coating is a continuous fiber of infinite length in principle of production, but it is preferable that the fiber have a length of at least 100 times or more of the thickness of the fiber. In the present specification, a fiber having a length of 100 times or more of the thickness of the fiber is defined as a "continuous fiber". The coating produced by the electrostatic spraying method is preferably a porous discontinuous coating composed of a deposit of continuous fibers. Such a form of coating not only can be handled as a single sheet as an aggregate, but also has the advantage that it has a very soft texture, and it is hard to be separated even when a shearing force is applied, and it is excellent in the followability to the body movement. Further, advantageously, the coating can be easily completely removed. In contrast, a continuous coating without pores is not easy to peel off and has a low sweat dissipation property, so that there is a fear that the skin will become blistered. In addition, it is difficult to completely remove the porous discontinuous coating formed of the aggregate of particles without damaging the skin, for example, an operation of applying friction or the like to the entire coating is necessary in order to completely remove the coating.

In the electrostatic spraying method using the electrostatic spraying device 10, the spraying composition which has been electrostatically sprayed into a fibrous state directly reaches the skin in a state in which the component (b) and the component (c) are charged while the component (a) evaporates. Since the skin is also charged as described above, the fibers adhere to the skin in the form of a single membrane by electrostatic forces. Since fine unevenness such as texture are formed on the surface of the skin, the fibers are more closely adhered to the surface of the skin in the form of a single membrane in combination with the anchoring effect due to the irregularities. When the electrostatic spraying is completed in this manner, the power of the electrostatic spraying device 10 is turned off. As a result, the electric field between the nozzle and the skin disappears, and the surface of the skin is immobilized with an electric charge. As a result, the adhesivity of the coating in a form of a single film is further elevated, the coating is hard to peel off from the time of wearing is difficult, and durability during use is improved. In addition, since the fiber constituting the coating contains moisture, there is no discomfort in the coating, an excellent moisturizing effect and a softening effect of the coating can be obtained, and the coating can be sufficiently adhered to the skin. The reason for this is considered to be that the fiber itself is softened by the plasticizing effect by the presence of moisture on the surface of the fiber or between the fibers to enhance the followablity to the fine concavo-convex surface, and that the moisture bleeds out on the fiber surface to cause liquid cross-linking between the fibers and the skin. Furthermore, since the coating has a moisture bearing coating in which moisture is present between fibers of the fibers constituting the coating or on the surface of the fibers, the fibers constituting the coating are hard to reflect light, the appearance of the coating tends to be transparent, and the skin can be covered in a natural appearance. These effects are further enhanced by the application of the liquid agent described hereafter.

The distance between the nozzle and the skin also depends on the voltage applied to the nozzle, but in order to successfully form the coating, it is preferable to be 10 mm or more and 160 mm or less, more preferable to be 20 mm or more and 150 mm or less, and even more preferable to be 40 mm or more and 150 mm or less. The distance between the nozzle and the skin can be measured by a commonly used non-contact sensor or the like.

Whether or not the coating formed by the electrostatic spraying method contains fibers, the basis weight of the coating is preferably 0.1 g/m$^2$ or more, more preferably 1 g/m$^2$ or more. Further, it is preferable to be 50 g/m$^2$ or less, and more preferable to be 40 g/m$^2$ or less. For example, the basis weight of the coating is preferably 0.1 g/m$^2$ or more and 50 g/m$^2$ or less, and more preferably 1 g/m$^2$ or more and 40 g/m$^2$ or less. By setting the basis weight of the coating in this manner, the adhesivity of the coating can be improved.

Note that the electrostatic spraying step of forming a coating by electrostatically spraying the composition directly onto the skin or nail means a step of forming a coating by electrostatically spraying onto the skin or nail. The step of electrostatically spraying the composition onto a location other than the skin or nail to produce a sheet of fibers and applying the sheet to the skin or nail differs from the above electrostatically spraying step.

In the present invention, before or after the electrostatic spraying step of forming a coating on the skin or nail by electrostatic spraying as described above, the step of applying a liquid agent other than the spraying composition to the skin, for example, by means other than electrostatic spraying, the composition containing one or more selected from a polyol in a liquid state at 20°C and an oil in a liquid state at 20°C may be carried out. By performing the application step of the liquid agent, the coating formed by the electrostatic spraying step becomes easily familiar to the application site, and the coating can be made highly adherent to the skin, and can be made extremely transparent. For example, a space is less likely to occur between the edge of the coating and the skin, thereby improving the adhesivity between the coating and the skin. As a result, peel, tearing, or the like of the coating is less likely to occur. In a more preferable embodiment, when the coating is a porous coating composed of a fiber deposit, the adhesivity with the skin is high despite the high porosity, and a large capillary force is apt to occur. Further, when the fibers are fine, it is easy to make the porous coating have a high specific surface area.

In particular, after forming a porous coating composed of a fiber deposit in the electrostatic spraying process, an application step of a liquid agent is performed to form a coating in which the liquid agent is present between the fibers forming the porous coating and/or on the surface of the fibers . This improves the coating adhesivity and maintains or improves the transparency of the coating for visual inspection. In particular, when the coating is colorless and transparent or colored and transparent, the coating becomes more difficult to visually recognize, so that the coating can appear like natural skin. In addition, when the coating is colored and transparent, the coating has a transparent feeling, so that it can appear as a part of the skin.

Examples of the oil in a liquid state at 20°C include a linear or branched hydrocarbon oil such as liquid paraffin, light isoparaffin, liquid isoparaffin, squalane, squalene; an ester oil such as amonoalcohol fatty acid ester, a monoglycerol fatty acid ester, a triglycerol fatty acid ester, a polyvalent alcohol fatty acid ester; a silicone oil such as dimethylpolysiloxane, dimethylcyclopolysiloxane, methylphenylpolysiloxane, methylhydrogenpolysiloxane, higher alcohol-modified organopolysiloxanes; a higher alcohol. Among these, a hydrocarbon oil and an ester oil are preferable from the viewpoint of smoothness etc. at the time of application. In addition, a vegetable oil such as an olive oil and a jojoba oil containing multiple kinds of hydrocarbon oils and ester oils, an animal oil such as liquid lanolin, and the like can be contained, or one or more kinds of liquid oils selected from the above-mentioned oils in a liquid at 20°C can be used in combination.

Examples of the hydrocarbon oil include liquid paraffin, squalane, squalene, n-octane, n-heptane, cyclohexane, light isoparaffin, liquid isoparaffin, and the like, and liquidparaffin and squalane are preferable from the viewpoint of impression from use. In addition, from the viewpoint of adhering the electrostatically sprayed coating film to the skin, the viscosity of the hydrocarbon oil at 30°C is preferably 10 mPa·s or more, more preferably 30 mPa·s or more. From this viewpoint, the total content of isododecane, isohexadecane, hydrogenated polyisobutene having viscosities of less than 10 mPa·s at 30°C is preferably 10 mass% or less, more preferably 5 mass% or less, even more preferably 1 mass% or less, even more preferably 0.5 mass% or less, and may not be contained.

Similarly, from the viewpoint of adhering the electrosprayed coating to the skin, the viscosity of the ester oil and the silicone oil at 30°C is preferably 10 mPa·s or more, more preferably 30 mPa·s or more.

The viscosity here is measured at 30°C with a BM-type viscometer (manufactured by Tokimek Corporation, measurement conditions: Rotor No.1, 60 rpm, 1 minute).

From the same viewpoint, the total content of the ether oil such as cetyl-1,3-dimethylbutyl ether, dicapryl ether, dilauryl ether, diisostearyl ether and the like in the liquid agent is preferably 10 mass% or less, more preferably 5 mass% or less, and even more preferably 1 mass% or less.

Examples of the ester oil include an ester consisting of a linear or branched chain fatty acid and a linear or branched chain alcohol or a polyvalent alcohol. Specifically, they include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethyleneglycol di(2-ethylhexanoate), dipentaerythritol fatty acid ester, N-alkylglycol monoisostearate, neopentylglycol dicaprate, diisostearyl malate, glyceryl di(2-heptylundecanoate), trimethylolpropane tri(2-ethylhexanoate), trimethylolpropane triisostearate, pentaerythritol tetra(2-ethylhexanoate), glyceryl tri(2-ethylhexanoate), trimethylolpropane triisostearate, cetyl 2-ethylhexanoate, 2-ethylhexyl palmitate, diethylhexyl naphthalenedicarboxylate, (C12-15)alkyl benzoate, cetearyl isononanoate, caprylic/capric triglyceride, dicaprylic/capric butyleneglycol, glyceryl trilaurate, glyceryl trimyristate, glyceryltripalmitate, glyceryl triisostearate, glyceryl tri(2-heptylundecanoate), glyceryl tribehenate, glyceryl tricocoate, castor oil fatty acid methyl ester, oleyl oleate, 2-heptylundecyl palmitate, diisobutyl adipate, N-lauryol-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di(2-ethylhexyl) succinate, triethyl citrate, 2-ethylhexyl para-methoxycinnamate, tripropylene glycol dipivalate.

Among these, from the viewpoint of adhering the coating, produced by electrostatic spraying, to the skin and in terms of excellent feeling of the coating upon application to the skin, at least one selected from the group consisting of octyldodecyl myristate, myristyl myristate, isocetyl stearate, isocetyl isostearate, cetearyl isonanoate, diisobutyl adipate, di-2-ethylhexyl sebacate, isopropyl myristate, isopropyl palmitate, diisostearyl malate, neopentylglycol dicaprate, (C12-15)alkyl benzoate, and caprylic/capric triglyceride, is preferable. At least one selected from the group consisting of isopropyl myristate, isopropyl palmitate, diisostearyl malate, neopentylglycol dicaprate, (C12-15)alkyl benzoate, and caprylic/capric triglyceride is more preferable. It is further preferable to contain one or more selected from neopentylglycol dicaprate, (C12-15)alkyl benzoate, caprylic/capric triglyceride and isopropyl myristate.

As the triglyceride, a fatty acid triglyceride is preferable, and the triglyceride is included in, for example, an olive oil, a jojoba oil, a macademia nut oil, a medform oil, a castor oil, a safflower oil, a sunflower oil, an avocado oil, a canola oil, a ginseng oil, a rice germ oil, and a rice bran oil.

Examples of the higher alcohol include a liquid C12-20 higher alcohol, specifically isostearyl alcohol, oleyl alcohol, and the like.

Examples of the preservative include phenoxyethanol, methyl para-hydroxybenzoate, ethyl para-aminobenzoate, isobutyl para-hydroxybenzoate, isopropyl para-hydroxybenzoate, ethyl para-hydroxybenzoate, butyl para-hydroxybenzoate, propyl para-hydroxybenzoate, benzyl para-hydroxybenzoate, ethylhexanediol.

Examples of the silicone oil include dimethylpolysiloxane, dimethylcyclopolysiloxane, methylphenylpolysiloxane, methylhydrogenpolysiloxane, a higher alcohol modified organopolysiloxane.

The kinematic viscosity of the silicone oil at 25°C is preferably 3mm$^2$ per second, more preferably 4 mm$^2$ per second, more preferably 5 mm$^2$ per second or more, more preferably 30 mm$^2$ per second or less, more preferably 20 mm$^2$ per second or less, and even more preferably 10 mm$^2$ per second or less, from the viewpoint of adhering the coating, produced by electrostatically spraying, to the skin.

Among these, it is preferable to contain dimethylpolysiloxane from the viewpoint of adhering the electrostatically sprayed coating to the skin.

The content of the liquid oil in the liquid agent is preferably 0.1 mass% or more, more preferably 0.5 mass% or more, and even more preferably 5 mass% or more. It is preferably 100 mass% or less. The content of the liquid oil in the liquid agent is preferably 0.1 mass% or more and 100 mass% or less, more preferably 0.5 mass% or more and 100 mass% or less.

Here, the HLB value of the liquid oil is preferably 10 or less, more preferably 8 or less, and even more preferably 6 or less. The HLB value is an index indicating the balance (Hydrophile-Lipophile Balance) between hydrophilicity and lipophilicity, and in the present invention, the HLB value is calculated by the following formula by Oda and Teramura et al.

$$\mathtt{HLB=(\Sigma\ inorganic\ value/\Sigma\ organic\ value)\ \times\ 10}$$

Examples of the polyol in a liquid state at 20°C and which is used in the liquid agent include an alkylene glycol such as ethylene glycol, propylene glycol, 1,3-propanediol, 1,3-butanediol; a polyalkylene glycol such as diethylene glycol, dipropylene glycol, polyethylene glycol having a weight average molecular weight of 2,000 or less, polypropylene glycol; a glycerin such as glycerin, diglycerin, triglycerin. Among these, ethylene glycol, propylene glycol, 1,3-butanediol, dipropylene glycol, polyethylene glycol having a weight average molecular weight of 2,000 or less, glycerin, and diglycerin are preferable from the viewpoint of the feeling of use such as smoothness upon application, and further propylene glycol, 1,3-butanediol, and glycerin are more preferable, and propylene glycol and 1,3-butanediol are more preferable.

It is preferable that the liquid agent have a viscosity of 5,000 mPa·s or less at 25°C from the viewpoint of improving the adhesivity between the coating formed by the electrostatic spraying method and the application site. The method of measuring the viscosity of the liquid is as described above.

Various methods can be used to apply the liquid agent to the skin. For example, by applying a liquid agent to the skin by a method such as dropping or sprinkling, and by providing a step of spreading the liquid agent, it becomes possible to conform to the skin or the coating, and a thin layer of the liquid agent can be formed. The step of spreading the liquid agent may employ, for example, a method such as rubbing using a finger of the user or a tool such as an applicator. The liquid agent may be simply dropped or sprinkled, but by providing the spreading step, it becomes possible to conform to the skin or the coating, and the coating adhesivity can be sufficiently improved. Alternatively, the liquid agent can be sprayed onto the skin to form a thin layer of the liquid agent. In this case, separate spreading is not particularly necessary, but the spreading operation after spraying is not hindered. When a liquid is applied after the formation of the coating, a sufficient amount of the liquid is applied to the skin, and the excess liquid can be removed by subjecting into contact with a sheet material the area where the liquid is applied, so that the excess liquid can be removed.

The amount of the liquid agent to be applied to the skin may be an amount necessary and sufficient to improve the adhesivity between the skin and the coating. When a liquid oil is contained in the liquid agent, from the viewpoint of ensuring the adhesivity between the skin and the coating, the amount of the liquid agent to be applied to the skin is such that the basis weight of the liquid oil is preferably 0.1 g/m$^2$ or more, more preferably 0.2 g/m$^2$ or more; preferably 40 g/m$^2$ or less, and more preferably 35 g/m$^2$ or less. For example, the amount of the liquid to be applied to the skin is such that the basis weight of the liquid oil is preferably 0.1 g/m$^2$ or more and 40 g/m$^2$ or less, more preferably 0.2 g/m$^2$ or more and 35 g/m$^2$ or less.

In addition, the amount of the liquid agent to be applied to the skin or the coating film is preferably 5 g/m² or more, more preferably 10 g/m² or more, more preferably 15 g/m² or more, preferably 50 g/m² or less, and more preferably 45 g/m² or less, from the viewpoint of improving the adhesivity between the skin and the coating film and improving the transparency.

Although the present invention has been described above with reference to its preferred embodiment, the present invention is not limited to the embodiments described above. For example, in the embodiment described above, a person who wants to form a coating on his/her own skin holds the electrostatic spraying device 10 and generates an electric field between the device 10 and his/her skin, but as long as an electric field is generated between the two, it is not necessary for him or her to hold the electrostatic spraying device 10.

With respect to the above embodiments, the present invention further discloses the following methods.

<1> A method for producing a coating comprising a step of directly electrostatically spraying a composition A onto human skin or nail, the composition A comprises components (a), (b) and (c):

(a) one or more volatile substances selected from the group consisting of an alcohol and a ketone,
(b) a water-insoluble polymer for fiber formation, and
(c) 0.2 mass% or more and 25 mass% or less of water,

wherein the mass ratio of component (b) to component (c), ((b)/(c)), is 0.4 or more and 50 or less.

<2> The method according to <1>, wherein the mass ratio of component (a) to component (c) in the composition A, ((a)/(c)), is 3 or more and 300 or less.

<3> The method according to <1> or <2>, which forms a coating of wet fibers on the surface of human skin or nail.

<4> The method according to any one of <1> to <3>, wherein the electrostatic spraying device is an electrostatic spraying device holdable by a human hand, or an electrostatic spraying device having an operation unit equipped with a spraying nozzle holdable by a human hand.

<5> The method according to any one of <1> to <4>, wherein the (a) volatile substance has a vapor pressure at 20°C of 0.01 kPa or more and 106.66 kPa or less, more preferably 0.13 kPa or more and 66.66 kPa or less, further more preferably 0.67 kPa or more and 40.00 kPa or less, and even more preferably 1.33 kPa or more and 40.00 kPa or less.

<6> The method according to any one of <1> to <5>, wherein the (a) volatile substance is preferably an alcohol, more preferably one or more selected from the group consisting of a monovalent chain aliphatic alcohol, a monovalent cyclic aliphatic alcohol, and a monovalent aromatic alcohol, and more preferably one or more selected from the group consisting of ethanol, isopropyl alcohol, butyl alcohol, phenylethyl alcohol, propanol and pentanol.

<7> The method according to any one of <1> to <6>, wherein the (a) volatile substance is preferably one or more selected from the group consisting of ethanol, isopropyl alcohol, and butyl alcohol, more preferably one or two selected from the group consisting of ethanol and butyl alcohol, and even more preferably a volatile substance comprising ethanol.

<8> The method according to any one of <1> to <7>, wherein component (a) is preferably a volatile substance comprising ethanol, and the content of ethanol in the component (a) is more preferably 80 mass% or more.

<9> The method according to any one of <1> to <8>, wherein the content of component (a) in the composition A is preferably 50 mass% or more, more preferably 55 mass% or more, further more preferably 60 mass% or more, even more preferably 65 mass% or more; preferably 95 mass% or less, more preferably 94 mass% or less, further more preferably 93 mass% or less, even more preferably 92 mass% or less; the content of component (a) in the composition is preferably 50 mass% or more and 95 mass% or less, more preferably 55 mass% or more and 94 mass% or less, further more preferably 60 mass% or more and 93 mass% or less, and even more preferably 65 mass% or more and 92 mass% or less.

<10> The method according to any one of <1> to <9>, wherein the content of the component (a) in the composition A is preferably 60 mass% or more and 92 mass% or less.

<11> The method according to any one of <1> to <10>, wherein the (b) water-insoluble polymer for fiber formation is a substance that can be dissolved in the (a) volatile substance and is a water-insoluble polymer.

<12> The method according to any one of <1> to <11>, wherein component (b) is one or more water-insoluble polymers selected from the group consisting of a fully saponified polyvinyl alcohol, a partially saponified polyvinyl alcohol, an oxazoline-modified silicone, a polyvinyl acetal diethylamino acetate, Zein, polyester, a polylactic acid, a polyacrylonitrile resin, a polymethacrylic acid resin, a polystyrene resin, a polyvinyl butyral resin, a polyethylene terephthalate resin, a polybutylene terephthalate resin, a polyurethane resin, a polyamide resin, a polyimide resin, a polyamide-imide resin; more preferably selected from the group consisting of a fully saponified polyvinyl alcohol, a partially saponified polyvinyl alcohol, a polyvinyl butyral resin, a polyurethane resin, a polymethacrylic acid resin, polyvinyl acetal diethylamino acetate, a poly(N-propanoylethyleneimine) graft-dimethylsiloxane/γ-aminopropylmethylsiloxane copolymer, polylactic acid, Zein; further more preferably a polyvinyl butyral resin; and even more preferably a water-insoluble resin at least containing a polyvinyl butyral resin.

<13> The method according to any one of <1> to <12>, wherein component (b) is a water-insoluble resin comprising a polyvinyl butyral resin.

<14> The method according to any one of <1> to <13>, wherein the content of component (b) in the composition A is preferably 1 mass% or more, more preferably 2 mass% or more, further more preferably 3 mass% or more, even more preferably 5 mass% or more; preferably 30 mass% or less, more preferably 25 mass% or less, further more preferably 20 mass% or less; the content of component (b) in the composition A is preferably 1 mass% or more and 25 mass% or less, more preferably 2 mass% or more and 25 mass% or less, further more preferably 3 mass% or more and 20 mass% or less, and even more preferably 5 mass% or more and 20 mass% or less.

<15> The method according to any one of <1> to <14>, wherein the content of component (b) in the composition A is preferably 5 mass% or more and 20 mass% or less.

<16> The method according to any one of <1> to <14>, wherein the content of component (b) in the composition A is preferably 7 mass% or more and 17 mass% or less.

<17> The method according to any one of <1> to <16>, wherein the content of the (c) water in the composition A is preferably 0.3 mass% or more, more preferably 0.35 mass% or more, even more preferably 0.4 mass% or more; preferably 20 mass% or less, more preferably 19 mass% or less, even more preferably 18 mass% or less; preferably 0.3 mass% or more and 20 mass% or less, more preferably 0.35 mass% or more and 19 mass% or less, and even more preferably 0.4 mass% or more and 18 mass% or less.

<18> The method according to any one of <1> to <17>, wherein the content of the (c) water in the composition A is preferably 0.4 or more and 18 or less.

<19> The method according to any one of <1> to <18>, wherein the mass ratio of component (b) to component (c) in the composition A, ((b)/(c)), is preferably 0.5 or more, more preferably 0.55 or more, further more preferably 0.6 or more; preferably 45 or less, more preferably 40 or less; preferably 0.5 or more and 45 or less, more preferably 0.55 or more and 40 or less, and even more preferably 0.6 or more and 40 or less.

<20> The method according to any one of <1> to <18>, wherein the mass ratio of component (b) to component (c) in the composition A, ((b)/(c)), is preferably 0.6 or more and 40 or less.

<21> The method according to any one of <1> to <20>, wherein the mass ratio of component (a) to component (c) in the composition A, ((a)/(c)), is preferably 3 or more and 300 or less; preferably 3 or more, more preferably 3.5 or more, more preferably 4 or more; preferably 300 or less, more preferably 250 or less, further more preferably 210 or less; preferably 3 or more and 300 or less, more preferably 3.5 or more and 250 or less, and even more preferably 4 or more and 210 or less.

<22> The method according to any one of <1> to <20>, wherein the mass ratio of component (a) to component (c) in the composition A, ((a)/(c)) is preferably 4 or more and 210 or less.

<23> The method according to any one of <1> to <22>, wherein the mass ratio of component (b) to component (a) in the composition A, ((b)/(a)), is preferably 0.02 or more, more preferably 0.025 or more, further more preferably 0.04 or more, even more preferably 0.07 or more, further even more preferably 0.08 or more; preferably 0.6 or less, more preferably 0.4 or less, further more preferably 0.3 or less, even more preferably 0.25 or less.

<24> The method according to any one of <1> to <22>, wherein the mass ratio of component (b) to component (a) in the composition A, ((b)/(a)), is 0.08 or more and 0.25 or less.

<25> The method according to any one of <1> to <24>, wherein the composition A further comprises another component selected from the group consisting of a conductivity controlling agent, a water-soluble polymer, a plasticizer of component (b), a coloring pigment, a constitutive pigment, a dye, a perfume, a repellent, an antioxidant, a stabilizer, a preservative, and various vitamins, and the content of the other component (s) is preferably 0.1 mass % or more and 30 mass % or less, and more preferably 0.5 mass % or more and 20 mass % or less.

<26> The method according to any one of <1> to <25>, wherein the viscosity of the composition A at 25°C is preferably from 2 to 3, 000 mPa·s, preferably 10 mPa·s or more, more preferably 20 mPa·s or more, even more preferably 30 mPa·s or more; preferably 1,500 mPa·s or less, more preferably 1,000 mPa·s or less, further more preferably 800 mPa·s or less; preferably 2 mPa·s or more and 3,000 mPa·s or less, more preferably 10 mPa·s or more and 1,500 mPa·s or less, further more preferably 20 mPa·s or more and 1,000 mPa·s or less, and even more preferably 30 mPa·s or more and 800 mPa·s or less.

<27> The method according to any one of <1> to <25>, wherein the viscosity of the composition A at 25°C is 15 mPa·s or more and 800 mPa·s or less.

<28> The method according to any one of <1> to <27>, wherein the electrostatic spraying is performed by using an electrostatic spraying device having a container containing the composition A, a nozzle for discharging the composition A therefrom, a supply device for supplying the composition A contained in the container to the nozzle, and a power supply for applying a voltage to the nozzle.

<29> The composition according to any one of <1> to <28>, wherein

the electrostatic spraying is performed by using an electrostatic spraying device,

the electrostatic spraying device comprises a nozzle,

the nozzle consists of a conductive material, and a non-conductive material surrounding the conductive material, and has a shape enabling the discharge of the composition A from the tip thereof.

<30> The composition according to any one of <1> to <29>, wherein

the electrostatic spraying is performed by using an electrostatic spraying device,

the electrostatic spraying device includes a nozzle and a housing,

the nozzle is disposed at one longitudinal end of the housing,

the nozzle is arranged on the housing such that the blowing direction of the composition coincides with the longitudinal direction of the housing and is convex toward the skin side.

<31> The method according to any one of <1> to <30>, wherein the sprayed composition is volatilized from droplets of a volatile substance as a solvent to solidify a polymer having a coating formability as a solute, and a fiber is formed by elongation deformation by a potential difference.

<32> The method according to any one of <1> to <31>, wherein the electrostatic spraying is performed by using an electrostatic spraying device,

the electrostatic spraying device comprises a nozzle,

the manufacturing method described in any one of <1> or <31>, in which the distance between the nozzle and the skin is preferably 10 mm or more and 160 mm or less, more preferably 20 mm or more and 150 mm or less, even more preferably 40 mm or more and 150 mm or less.

<33> The method according to any one of <1> to <32>, wherein the basis weight of the coating formed by the electrostatic spraying be 0.1 g/m$^2$ or more, 1 g/m$^2$ or more, 50 g/m$^2$ or less, 40 g/m$^2$ or less, 0.1 g/m$^2$ or more and 50 g/m$^2$ or less, and 1 g/m$^2$ or more and 30 g/m$^2$ or less.

<34> The method according to any one of <1> to <33>, wherein the content of glycol in the composition is 10 mass% or less, more preferably 3 mass% or less, more preferably 1 mass% or less, or substantially free of glycol.

<35> The method according to any one of <1> to <34>, wherein component (a) is a volatile substance at least comprising ethanol and component (b) is a polyvinyl butyral resin.

<36> The method according to any one of <1> to <35>, wherein component (a) is a volatile substance at least comprising ethanol, and the content of glycol is 10 mass% or less.

<37> The method according to any one of <1> to <36>, wherein the component (a) is a volatile substance at least comprising ethanol, the content of ethanol in the volatile substance is 50 mass% or more and 100 mass%, and the content of glycol is mass% or less.

<38> A spraying composition for use in electrostatically spraying directly onto a human skin or nail to produce a coating comprising fibers on the surface of the skin or nail by means of an electrostatically spraying device holdable by a human hand or an electrostatically spraying device comprising an operation unit comprising a spraying nozzle holdable by a human hand, the composition comprising the following components (a), (b) and (c) :

    (a) a volatile substance comprising ethanol,
    (b) a water-insoluble polymer for fiber formation, and
    (c) 0.2% to 25 mass% of water,

wherein the mass ratio of component (b) to component (c), ((b) / (c)), is 0.4 or more and 50 or less.

<39> The spraying composition according to <38>, wherein component (b) is one or more water-insoluble polymers selected from the group consisting of a fully saponified polyvinyl alcohol, a partially saponified polyvinyl alcohol, a polyvinyl butyral resin, a polyurethane resin, and a polymethacrylic acid resin.

<40> The spraying composition according to <38>, wherein the component (b) is a polyvinyl butyral resin.

<41> The spraying composition according to any one of <38> to <40>, wherein the electrostatic spraying device includes a refillable cartridge, the spraying composition is contained in the container of the refillable cartridge.

<42> The spraying composition according to any one of <38> to <41>, wherein the refillable cartridge is detachably used in the electronic spraying device, comprising a container, and the spraying composition is contained in the container, and the mist composition is a refillable castle-containing mist composition as described in any of <38> to <41>.

<43> The spraying composition or the spraying composition containing a cartridge according to <41> or <42>, wherein the refillable cartridge further comprises a nozzle.

<44> The spraying composition or the spraying composition in a cartridge according to <43>, wherein the nozzle of the refillable cartridge comprises an electrode.

<45> The spraying composition according to any one of <41> to <44>, wherein the volume of the container of the refillable cartridge is 1 mL or more and 25 mL or less, more preferably 1.5 mL or more and 20 mL or less, and even more preferably 2 mL or more and 15 mL or less.

<46> The spraying composition according to any one of <38> to <45>, wherein the mass ratio of component (a) to component (c) in the spraying composition, ((a)/(c)), is 3 or more and 300 or less.

<47> The spraying composition according to any one of <38> to <45>, wherein the mass ratio of component (a) to component (c) in the spraying composition, ((a)/(c)), is 4 or more and 210 or less.

<48> The spraying composition according to any one of <38> to <47>, wherein the spraying composition has viscosity at 25°C of 2 mPa·s or more and 3,000 mPa·s or less.

<49> The spraying composition according to any one of <38> to <47>, wherein the spraying composition has a viscosity at 25°C of 15 mPa·s or more and 1,000 mPa·s or less.

<50> The spraying composition according to any one of <38> to <49>, wherein the content of component (a) is 50 mass% or more and 95 mass% or less; the content of component (b) is 1 mass% or more and 30 mass% or less, and the content of the component (c) is 0.3 mass% or more and 20 mass% or less in the spraying composition.

<51> The spraying composition according to any one of <38> to <49>, wherein the content of component (a) is 60 mass% or more and 93 mass% or less, the content of component (b) is 5 mass% or more and 20 mass% or less, and the content of component (c) is 0.4 mass% or more and 18 mass% or less in the spraying composition.

<52> The spraying composition according to any one of <38> to <50>, wherein the mass ratio of component (b) to component (a) in the spraying composition, ((b)/(a)), is 0.07 or more and 0.3 or less.

<53> The spraying composition according to any one of <38> to <50>, wherein the mass ratio of component (b) to component (a) in the spraying composition, ((b)/(a)), is 0.08 or more and 0.25 or less .

<54> The spraying composition according to any one of <38> to <51> wherein the mass ratio of component (b) to component (c) in the spraying composition, ((b)/(c)), is 0.6 or more and 40 or less.

[Examples]

The present invention will be explained in more detail by means of the following examples. However, the scope of the present invention is not limited to these examples. Unless otherwise specified, "%" means "mass%".

[Example 1]

(1) Preparation of spraying compositions

Ethanol (trade name: Ethanol (99.5%) manufactured by Wako Pure Chemical Industries, Ltd.) was used as component (a) of the spraying composition. Polyvinyl butyral or partially saponified polyvinyl alcohol, which will be described hereafter, was used as component (b) of the spraying composition. Ion-exchanged water was used as component (c) of the spraying composition. Component (a), component (b), component (c), and other component (d) glycerin were weighed into a glass container so as to have the amounts shown in Table 1, and stirred at room temperature for about 12 hours using a magnetic stirrer to obtain a uniformly transparent solution. Examples 1 to 13 and Comparative Examples 1 to 2 were spraying compositions.

(2) Electrostatic spraying step

Using an electrostatic spraying device 10 having the configuration shown in FIG. 1 and having the external appearance shown in FIG. 7, an electrostatic spraying method was performed for 60 seconds on skin models cut out to 50 mm × 50 mm (artificial leather, protein leather PBZ13001BK, manufactured by Idemitsu Technofine Co., Ltd.). The conditions of the electrostatic spraying method were as follows.

- Applied voltage: 30 kV
- Distance between nozzle and skin: 130 mm
- Volume of spraying composition discharged: 4 mL/h
- Environment: 25°C, 30%RH

This electrostatic spraying resulted in the formation of a single film of a fiber deposit on the surface of the skin model.
The polyvinyl butyral used in Examples and Comparative Examples is as follows.

PVB-1: polyvinyl butyral (trade name: S-LEC BM-1, manufactured by Sekisui Chemical Co., Ltd.)
PVB-2: polyvinyl butyral (trade name: S-LEC KS-1, manufactured by Sekisui Chemical Co., Ltd.)
PVB-3: polyvinyl butyral (trade name: S-LEC BH-3, manufactured by Sekisui Chemical Co., Ltd.)
PVA: partially saponified polyvinyl alcohol (manufactured by Nippon Vignette Povar Co., Ltd.: trade name JMR-150L)

<Evaluation>

Regarding the coatings formed in Examples and Comparative Examples, the coating formability, coating adhesivity, and coating durability were evaluated based on the following criteria. The results are given in Table 1. The evaluation of adhesivity and durability of the coating was carried out within 30 minutes after the coating was formed.

<Coating Formability>

The discharge state in the electrostatic spraying process and the appearance of the formed coating were visually evaluated, and the coating formability was evaluated based on the following criteria.

A: Fibrous electrospinning composition is discharged stably for 1 minute, and a circular uniform coating is formed.
B: Particulate electrostatic spinning composition is discharged stably for 1 minute to form a circular uniform coating.
C: A fibrous or particulate electrostatic spinning composition is discharged stably for 1 minute, a circular coating is formed, and the coating is somewhat uneven (liquid droplet defects in the coating are $\varphi$ 5 mm or less).
D: The spin composition is not discharged within 1 minute, or the fibrous or particulate electrospinning composition is not stably discharged and the coating is uneven.

<Coating Adhesivity >

Adhesivity was evaluated by the bending test of the skin model in which the coating was formed. The coating was placed on the outside, and the bending at 180 degrees was repeated 5 times, and the adhesiveness was evaluated on the basis of the adherence state of the coating and the skin model according to the following criteria.

A: No separation or peeling off was observed between the skin model and the membrane
B: The membrane is separated only within 10 mm from the bent portion, the coating is not peeled off.
C: Separation of the coating occurs from the bent portion over the circumference of 10 mm, but the coating is not peeled off.
D: The coating is completely peeled off

<Durability on the Skin>

The durability was evaluated by an abrasion test of the coating. The skin model was fixed and the coating was rubbed 5 times in one direction at a distance of 2 cm while a load of about 50 gf was applied from the top with the index finger. The durability was evaluated based on the following criteria based on the adhesivity state of the coating and the skin model and the adhesivity to the index finger.

A: Coating is not peeled, torn, or stuck to the index finger at all.
B: Coating is torn at the surface, but not stuck to the index finger.
C: Coating is torn at the surface, and stuck to the index finger.
D: Coating is completely peeled off.

[Table 1]

| | | Example 1 | Example 2 | Example 3 | Example 4 | Example 5 | Example 6 | Example 7 | Example 8 | Example 9 | Example 10 | Comparative Example 1 | Comparative Example 2 | Example 11 | Example 12 | Example 13 |
|---|---|---|---|---|---|---|---|---|---|---|---|---|---|---|---|---|
| Spraying Composition | Component (a) | Ethanol | Ethanol | Ethanol | Ethanol | Ethanol | Ethanol | Ethanol | Ethanol | Ethanol | Ethanol | Ethanol | Ethanol | Ethanol | Ethanol | Ethanol |
| | Content (mass %) | 89.5 | 87.5 | 85.0 | 83.0 | 72.0 | 70.0 | 85.0 | 85.0 | 65.0 | 93.0 | 55.0 | 53.0 | 72.0 | 85.5 | 87.0 |
| | Component (b) | PVB-1 | PVB-1 | PVB-1 | PVB-1 | PVB-1 | PVB-1 | PVB-2 | PVB-3 | PVB-1 | PVB-1 | PVB-1 | PVB-1 | PVA | PVB1 | PVB1 |
| | Content (mass %) | 10.0 | 10.0 | 10.0 | 10.0 | 10.0 | 10.0 | 10.0 | 10.0 | 30.0 | 2.0 | 10.0 | 10.0 | 10.0 | 14.0 | 8.0 |
| | Component (d) | - | Glycerol | - | Glycerol | - | Glycerol | - | - | - | - | - | Glycerol | - | - | - |
| | Content (mass %) | - | 2.0 | - | 2.0 | - | 2.0 | - | - | - | - | - | 2.0 | - | - | - |
| | Component (c) | Water | Water | Water | Water | Water | Water | Water | Water | Water | Water | Water | Water | Water | Water | Water |
| | Content (mass %) | 0.5 | 0.5 | 5.0 | 5.0 | 18.0 | 18.0 | 5.0 | 5.0 | 5.0 | 5.0 | 35.0 | 35.0 | 18.0 | 0.5 | 5.0 |
| | Viscosity (mPa·s) | 51.9 | 55.3 | 55.7 | 55.8 | 61.4 | 78.1 | 36.4 | 760 | 1209.6 | 14.1 | 100 | 120 | 33.7 | 141.8 | 17.0 |
| Coating Forms | Coating formability | A | A | A | A | A | A | A | A | C | B | D | D | C | A | A |
| | Coating Adhesivity | B | A | A | A | A | A | A | A | A | A | - | - | B | A | B |
| | Coating Durability | B | A | B | A | B | A | B | B | C | C | - | - | B | B | B |
| | b/c | 20.0 | 20.0 | 2.0 | 2.0 | 0.6 | 0.6 | 2.0 | 2.0 | 6.0 | 0.4 | 0.3 | 0.3 | 0.6 | 28.0 | 1.6 |
| | b/a | 0.11 | 0.11 | 0.12 | 0.12 | 0.14 | 0.14 | 0.12 | 0.12 | 0.46 | 0.022 | 0.18 | 0.19 | 0.14 | 0.16 | 0.09 |
| | a/c | 179 | 175 | 17 | 17 | 4 | 4 | 17 | 17 | 13 | 19 | 2 | 2 | 4 | 171 | 17 |

EP 3 613 405 A1

As is clear from Table 1, Examples 1 to 13 in which the content of component (c) is 30 mass% or less, and the mass ratio of component (b) to component (c) is 0.4 or more, all of which exhibit more excellent coating formability than Comparative Examples 1 and 2 in which the content of component (c) is 35 mass% and the mass ratio of component (b) to component (c) is 0.3. Furthermore, the balance between the (c) water and component (b) shows that Examples 1 to 8 exhibit higher coating formability and coating durability. Further, it is observed that Examples in which the viscosity is 10 mP·s or more and 1,000 mP·s or less exhibit more excellent coating formability, coating adhesiveness, and coating durability.

Note that under the same condition as the electrostatic spraying device 10 having the configuration shown in FIG. 1 and having the appearance shown in FIG. 7, with the electrostatic spraying device 10 having the configuration shown in FIG. 2 or the electrostatic spraying device 10 having the configuration shown in FIG. 5, the coating was successfully formed as with Examples shown in Tables 1 or 2.

[Description of Symbols]

| 10 | Electrostatic spraying device |
| 11 | Low-voltage power supply |
| 12 | High-voltage power supply |
| 13 | Auxiliary electrical circuit |
| 15A | Microgear pump |
| 14 | Container (accommodating portion)? |
| 15 | Nozzle |
| 15B | Pipe |
| 15C | Flexible pipe |
| 19 | Current-limiting resistor |
| 20 | Housing |

**Claims**

1. A method for producing a coating comprising fibers on the surface of skin or nail, comprising directly electrostatically spraying onto human skin or nail, the composition A comprising the following components (a), (b) and (c) :

   (a) one or more volatile substances selected from the group consisting of an alcohol and a ketone,
   (b) a water-insoluble polymer for fiber formation,
   (c) 0.2 mass% or more and 25 mass% or less of water;

   wherein the mass ratio of component (b) to component (c), ((b) / (c), is of 0.4 or more and 50 or less.

2. The method according to Claim 1, wherein the mass ratio of component (a) to component (c) in the composition A, ((a) / (c)), is 3 or more and 300 or less.

3. The method according to Claim 1 or 2, wherein the viscosity of the composition A at 25°C is 2 mPa·s or more and 3,000 mPa·s or less, preferably 20 mPa·s or more and 1,000 mPa·s or less.

4. The method according to any one of Claims 1 to 3, which forms a coating of wetted fibers on the surface of human skin or nail.

5. The method according to any one of Claims 1 to 4, wherein the electrostatic spraying is by means of an electrostatically spraying device holdable by a human hand or an electrostatically spraying device comprising an operation unit comprising a spraying nozzle holdable by a human hand.

6. The method according to any one of Claims 1 to 5, wherein component (a) in composition A is a volatile substance comprising ethanol, and the component (b) is a polyvinyl butyral resin.

7. The method according to any one of Claims 1 to 6, wherein the content of component (a) is 50 mass% or more and 95 mass% or less, the content of component (b) is 1 mass% or more and 30 mass% or less, and the content of component (c) is 0.3 mass% or more and 18 mass% or less in the composition A.

8. The method according to any one of Claims 5 to 7, wherein the electrostatic spraying device comprises a refillable cartridge filled with the composition A.

9. A spraying composition for use in electrostatically spraying a composition directly onto the human skin or nail to produce a coating comprising fibers on the surface of the skin or nail by means of an electrostatically spraying device holdable by a human hand or an electrostatically spraying device comprising an operation unit comprising a spraying nozzle holdable by a human hand, the following components (a), (b) and (c):

   (a) a volatile substance containing ethanol,
   (b) a water-insoluble polymer for fiber formation,
   (c) 0.2% to 25 mass% of water,

   wherein the composition contains a mass ratio of component (b) to component (c), ((b)/(c)), of 0.4 or more and 50 or less.

10. The spraying composition according to Claim 9, wherein the electrostatic spraying device comprises a refillable cartridge, and the spraying composition is filled in an accommodating portion of the refillable cartridge.

11. The spraying composition according to Claim 9, wherein the refillable cartridge is detachably used in the electrostatic spraying device, wherein the refillable cartridge comprises a container, and the spraying composition is contained in the container.

12. The spraying composition according to Claim 10 or 11, wherein the refillable cartridge further comprises a nozzle.

13. The spraying composition according to any one of Claims 9 to 12, wherein the mass ratio of component (a) to component (c) in the spraying composition, ((a)/(c)), is 3 or more and 300 or less.

14. The spraying composition according to any one of Claims 9 to 13, wherein the viscosity of the spraying composition at 25°C is 10 mPa·s or more and 1,500 mPa·s or less.

15. The spraying composition according to any one of Claims 9 to 14, wherein the content of component (a) in the spraying composition is 50 mass% or more and 95 mass% or less, the content of component (b) is 1 mass% or more and 30 mass% or less, and the content of component (c) is 0.3 mass% or more and 20 mass% or less.

[Figure 1]

[Figure 2]

[Figure 3]

[Figure 4]

[Figure 5]

[Figure 6]

[Figure 7]

## INTERNATIONAL SEARCH REPORT

| International application No. |
| --- |
| PCT/JP2018/016186 |

### A. CLASSIFICATION OF SUBJECT MATTER

Int. Cl. A61K8/02(2006.01)i, A45D40/00(2006.01)i, A61K8/34(2006.01)i, A61K8/35(2006.01)i, A61K8/81(2006.01)i, A61K8/84(2006.01)i, A61L26/00(2006.01)i, A61Q3/00(2006.01)i, A61Q19/00(2006.01)i, B05B5/025(2006.01)i, B05B11/00(2006.01)i, B05D1/04(2006.01)i

According to International Patent Classification (IPC) or to both national classification and IPC

### B. FIELDS SEARCHED

Minimum documentation searched (classification system followed by classification symbols)

Int. Cl. A61K8/02, A45D40/00, A61K8/34, A61K8/35, A61K8/81, A61K8/84, A61L26/00, A61Q3/00, A61Q19/00, B05B5/025, B05B11/00, B05D1/04

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| A | JP 2006-104211 A (THE PROCTER & GAMBLE CO.) 20 April 2006, claims, paragraphs [0013]-[0017], [0019]-[0022], [0047]-[0051], [0061] & US 6531142 B1, claims, column 4, line 12 to column 5, line 10, column 5, lines 14-66, column 9, line 38 to column 10, line 52, column 12, lines 55-66 & WO 2001/012139 A1 & EP 1204396 A1 & CN 1379657 A | 1-15 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 12.06.2018 | 26.06.2018 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
|---|
| PCT/JP2018/016186 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2006-095332 A (THE PROCTER & GAMBLE CO.) 13 April 2006, claims, paragraphs [0027]-[0030], [0035], [0036], [0045] & US 6514504 B1, claims, column 8, line 65 to column 10, line 66, column 13, line 1 to column 14, line 17, column 18, lines 27-54 & WO 2001/012137 A1 & EP 1204397 A1 & CN 1379656 A & KR 10-0459751 B1 | 1-15 |
| A | JP 2006-077031 A (THE PROCTER & GAMBLE CO.) 23 March 2006, claims, paragraphs [0012]-[0021], [0027]-[0033], [0062] & WO 2001/012138 A1, claims, page 6, line 4 to page 9, line 10, page 10, line 20 to page 12, line 20, page 20, lines 4-11 & US 7078046 B1 & EP 1204398 A1 & CN 1379658 A | 1-15 |
| A | JP 2003-507165 A (THE PROCTER & GAMBLE CO.) 25 February 2003, claims, paragraphs [0018], [0022] & US 6318647 B1, claims, page 2, line 62 to page 3, line 2, page 3, lines 32-38 & WO 2001/012335 A1 & EP 1202813 A1 & CN 1379701 A | 1-15 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004525272 A
- JP 2006095332 A
- JP 2007032534 A